(19) Europäisches Patentamt / European Patent Office / Office européen des brevets

(11) **EP 3 081 229 A1**

(12) **EUROPEAN PATENT APPLICATION**
published in accordance with Art. 153(4) EPC

(43) Date of publication:
**19.10.2016 Bulletin 2016/42**

(51) Int Cl.:
*A61K 39/00* (2006.01)     *G06F 19/00* (2011.01)

(21) Application number: **14830810.9**

(22) Date of filing: **02.12.2014**

(86) International application number:
**PCT/ES2014/070888**

(87) International publication number:
**WO 2015/082745 (11.06.2015 Gazette 2015/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.12.2013 ES 201331759**
**12.05.2014 EP 14382167**

(71) Applicants:
• **Universidad del Pais Vasco - Euskal Herriko Unibertsitatea (UPV/EHU)**
**48940 Leioa, Vizcaya (ES)**
• **Univerza na Primorskem, Universita' del Litorale**
**6000 Koper (SI)**

(72) Inventors:
• **LEGARRETA SOLAGUREN, Leire**
**E-48940 Leioa (Vizcaya) (ES)**
• **MARTÍNEZ FERNÁNDEZ, Luis**
**E-48940 Leioa (Vizcaya) (ES)**
• **MARTÍNEZ DE LA FUENTE MARTÍNEZ, Ildefonso**
**E-48940 Leioa (Vizcaya) (ES)**
• **MILANIC, Martin**
**6000 Koper (SI)**

(74) Representative: **Balder IP Law, S.L.**
**Paseo de la Castellana 93**
**5a planta**
**28046 Madrid (ES)**

(54) **METHOD FOR DESIGNING A VACCINE**

(57)     Computational procedure for designing a vaccine for a disease that starting:
- from a plurality of amino acid $S_1...S_k$ chains obtained from patients infected with such disease; and,
- from a T set of target epitopes, each epitope being an amino acid chain; comprises the steps of:
i) obtaining by means of a combinatory optimization method a plurality of amino acid chains as short as possible with respect to the combinatory optimization method used having as sub-chains at least a $\lambda$ integer of epitopes of the T set in each one of the $S_i$ sequences, $\lambda$ having a value such that the length of the resulting chain is long enough for eliciting an immune response, but not too long so as to elicit an autoimmune response;
ii) choosing from the plurality of chains obtained in the previous step at least one final immunogen candidate by at least one standard experimental method;
iii) developing the vaccine from said immunogen.

FIG. 1

**Description**

**TECHNICAL FIELD**

The present invention lies within the field of vaccine design, and more specifically the computational design of vaccines.

**BACKGROUND OF THE INVENTION**

It is known that in the presence of a virus, the immune systems develops proteins called antibodies which bind to viral parts called antigens. The antibody binds to one or more of the amino acid sequences of the antigen, called epitopes. The epitopes may be linear -consisting in consecutive amino acids within the primary structure of the antigen- or conformational -non-consecutive amino acids within the sequence, but located together in the folded structure-. Once the immune system develops an antibody for a virus, it is memorized and used to neutralize any future virus with the same epitopes. This is the underlying idea of vaccines, which are developed in order to mimic the epitopes of the virus. Unfortunately, viruses may mutate and the epitope sequence may change, thus avoiding being detected by antibodies.
The vast antigenic diversity of certain viral pathogens poses a great complexity for the development of vaccines. Furthermore, the identification of epitopes by conventional means is a cumbersome task which, usually, entails high economic costs.
In fact, the main obstacle to the successful development of most vaccines is derived from antigenic diversity, since the high mutability rate of many viruses and bacteria causes the epitopes not to be conserved over time and in the diffusion geographic space thereof.
The standard methods for obtaining vaccines are based in choosing immunogens based on its epidemiological presentation, which enables designing overlapping peptides along the chain of the protein molecule; first, they are synthetized and later tested in cytotoxicity assays along with other kind of tests. In most cases, these conventional means present limited effective results.
The computational design of vaccines is also known, which importance is increasing every year. In fact, the problem in designing a vaccine may be formulated in a combinatorial manner as the choice of an aminoacid sequence containing epitopes that maximize the efficacy of the antibodies against actual viruses. Some epitopes are more frequently produced than others in natural viral populations; therefore, a common approach is to maximize the coverage of epitopes appearing in the vaccine given a limit on their length, in the sense that the most common epitopes are more likely to be included.
There exist different techniques or methods to solve the problem. For example, Nickle (D.C. Nickle et al., "Coping with viral diversity in HIV vaccine design", PLoS Comput. Biol. 3:e75 (2007) 0754-0762) based their method in the search of the sequence in the center of the followed by the addition of a set of epitopes (COT+). Meanwhile, Fischer used genetic algorithms (Fisher W. et al, "Polyvalent vaccines for optimal coverage of potential T-cell epitopes in global HIV-1 variants", Nature Medicine 13(1) (2007) 100106), Toussaint used integer linear programming (Toussaint N.C. et al, "A mathematical framework for the selection of an optimal set of peptides for epitope-based vaccines", PLoS Comput. Biol. 4:e1000246 (2008) 1-10), and Gilles used a three rounds consensus (Gilles B.M. et al, "A computationally optimized broadly reactive antigen (COBRA) based H5N1 VLP vaccine elicits broadly reactive antibodies in mice and ferrets", Vaccine 29 (2011) 3043-3054, [Ref1]).
A key point in most of these computational techniques is to maximize the frequency of the epitopes appearing in the vaccine, in the sense that the most common epitopes are more likely to be included in the vaccine.
However, the formulations of the current optimization problems do not take on account several biological limitations or other physiological factors. A synthetic peptide (i.e., vaccine) must be biologically viable: it has to be cleaved, transported and presented, all of this in a correct manner. The delivery methods (through vectors) impose additional length restrictions. Recently, Kulkarni (Kulkarni et al, et al, "HIV-1 p24gag derived conserved element DNA vaccine increases the breadth of immune response in mice". PloS one, 8(3):e60245, 2013) argued that the idea of the optimized coverage may not be correct and that the inclusion of certain immunodominant epitopes may actually decrease the development of antibodies. Therefore, it is not yet clear what has to be optimized.

**DESCRIPTION OF THE INVENTION**

The invention relates to a procedure for designing a vaccine according to claim 1. Preferred embodiments of the invention are defined in the dependent claims.
In order to cope with the existing difficulties in the current processes for preparing vaccines, the present invention is based on designing the immunogen itself, which should be able to maximize the antigenic characteristics of vaccines, thus enabling the protein sequences generating the immune response to tend to effectively encompass the maximum possible variability of the virus. The procedure of the invention produces specific highly effective immunogens stimulating the immune response, which is critical for controlling the viremia during infection.

To this end, in the procedure for designing vaccines of the present invention, the balance that the coverage of epitopes in the vaccine with respect to the antigenic diversity of proteins is mathematically formulated, imposing as the starting point a specific level of balance in coverage (by means of the $\lambda$ parameter), which allows circumventing the antigenic diversity.

The invention relates to a computational procedure for designing a vaccine for a disease starting:

- from a plurality of amino acid $S_1,...,S_k$ chains obtained from patients infected with such disease; and,
- from a T set of target epitopes, each epitope being an amino acid chain.

The method comprises the steps of:

i) obtaining by means of a combinatory optimization method a plurality of amino acid chains as short as possible with respect to the combinatory optimization method used having as sub-chains at least a $\lambda$ integer of epitopes of the T set being sub-chains in each one of the $S_i$ chains, $\lambda$ having a value such that the length of the resulting chain is long enough for eliciting an immune response, but not too long so as to elicit an autoimmune response;

ii) choosing from the plurality of chains obtained in the previous step at least one final immunogen candidate by at least one standard experimental method; iii) developing the vaccine from said immunogen.

That is to say, in the procedure of the invention a specific level of balance in the coverage is established, quantified by the $\lambda$ parameter. This produces, as emerging property and added value, a good level of coverage.

The method of combinatorial optimization may be an integer programming method. In such case, the plurality of amino acid chains obtained have the shortest possible length, since by integer programming a global minimum is obtained.

Alternatively, a hill climbing algorithm, a genetic algorithm or a simulated annealing algorithm may be used as combinatorial optimization method.

In the case of a genetic algorithm, it preferably comprises:

- prefixing a n number of epitopes in the vaccine, a s size of the initial population of vaccine candidates, a m number of evolution steps, a pr_cross crossing probability and a pr_mut mutation probability;
- generating an initial population of s vaccine candidates wherein each member is obtained randomly concatenating an adequate number of chains selected among $S_1,...,S_k$ until obtaining s epitopes;
- selecting s vaccine candidates from the population based on their suitability and applying crossover and mutation operations, and replacing the population with the new obtained candidates;
- repeating the above process m times, and finally take the w candidate with better suitability obtained in the m evolution steps;
- obtaining a common superchain, with a next to the shortest length, of the epitopes belonging to w by a greedy algorithm.

In the case of the hill climbing algorithm a local minimum, not global, is obtained, so the plurality of amino acid chains obtained have the shortest possible length obtainable in an environment of the solution found, but not so short as if they were obtained by integer programming.

For each vaccine to be designed, such $S_1,...,S_k$ amino acid chains preferably correspond to the same protein for all infected patients, or two or more proteins corresponding to the $S_1,...,S_k$ aminoacid chains of infected patients may also be used.

Said T set of epitopes may be formed by all the chains of a determined length, or may be empirically obtained, or may be obtained from a base of epitopes.

Such standard experimental method preferably consists in applying immunogenicity tests for the final selection of the vaccine.

Said plurality of $S_1,...,S_k$ amino acid chains or sequences are preferably obtained from a database of biological sequences.

According to a preferred embodiment of the method of the invention, the steps i)-iii) are repeated for different $\lambda$ values and the one providing the highest epitope coverage is chosen from the solutions obtained.

The step of selecting at least one final immunogen candidate from the plurality of chains obtained in the previous step preferably comprises a reduced set formed by the candidates generating the highest antigenic response in cell cultures and, in the next step, selecting among the latter the candidate which most efficiently protects a group of animals infected with the target pathogen.

The step of developing a vaccine from said immunogen preferably comprises performing the three experimentation steps in humans and once finished and the relevant official authorization obtained, proceeding to its industrial production using genetically engineered bacteria and further labelling.

In the procedure of the present invention a new criterion in vaccine design complementing the criterion of obtaining a high coverage is presented, setting a combinatorial condition of imposing a certain equilibrium level for each viral sequence, which ensures that all the viral sequences cover at least a minimum number of epitopes. This restriction has a very interesting result: the frequencies of the epitopes in the vaccine are high. This leads to the desirable condition that the common epitopes are more likely to be covered in the vaccine. Furthermore, this combinatorial condition guarantees a better distribution of the covered epitopes in the target chains, which helps fighting against the capacity of the virus to escape of the immunological diversity.

Other advantages and additional characteristics of the invention will be apparent in light of the following detailed description and will be particularly pointed out in the appended claims.

**BRIEF DESCRIPTION OF THE DRAWINGS**

To complete the description and in order to aid a better understanding of the characteristics of the invention, according to a practical embodiment thereof, a set of figures has been attached as integral part of the description, in which by way of illustration and not limitation the following has been represented:

Figure 1 shows the length of the vaccine obtained as a function of the $\lambda$ parameter in the real application example using the procedure of the invention.

Figure 2 shows the coverage of the vaccine obtained as a function of the l length of the vaccine in the real application example using the procedure of the invention.

Figures 3, 4 and 5 show images of the three dimensional structures of the vaccine candidates obtained in examples 1, 2 and 3, respectively.

**DESCRIPTION OF AN EMBODIMENT OF THE INVENTION**

The procedure of computational vaccine design of the present invention is based on the problem of the shortest common superchain, in this case searching the shortest so-called $\lambda$-super-chain.

In the development of the present case, we started with a finite alphabet A. A* is denoted as the $A^* = U_{n=1}^{\infty} A^n \, |U \, \{\varepsilon\}$ set of all the finite chains or sequences (both terms are interchangeably used throughout the present description) of elements of A, where $\varepsilon$ denotes the empty chain.

It is said that a chain $s=(s_1, ..., s_n)$ is sub-chain of other chain $t=(t_1, ...,t_m)$, if there exists an index u in {1, ..., n-m+1} such that $t_{u+i-1}=s_i$ for each i in {1, ..., n}. That is to say, s is a sub-chain of t if t can be written as t = u+s+w for some chains u and w in A.

The problem is posed as follows:

- Be $S_1,...,S_k$ a set of chains of A*; be T a set of target chains; and be $\lambda$ a natural number, $\lambda \in N$.
- A $\lambda$-superchain is defined for $(S_1,..., S_k, T)$ as a chain $v \in A^*$ such that for each $i \in \{,...,k\}$ there is at least one $\lambda$ number of different chains in T and which are common subchains of both $S_i$ and v.

Given the input $S_1,...,S_k$ set of chains and a t target chain, $t \in T$, the frequency f of the chain t, f(t), is defined as the number of $S_i$ chains containing t, regardless of the number of times t may be expressed as a sub-chain of a given $S_i$ chain.

If v is a $\lambda$-superchain for $(S_1,..., S_k, T)$, it is defined as coverage of v, c(v), according to the following expression:

$$c(V) = \frac{\sum_{t \in T : t \text{ subchain of } v} f(t)}{\sum_{t \in T : t \text{ subchain of any } S_i} f(t)} \quad [1]$$

It may be proven that, as the $\lambda$ value is increased, the ratio c(v) gets increasingly closer to its maximum value 1. For the vaccine design procedure of the present invention it comes to look for the shortest possible $\lambda$-superchain. Thus, in the case of the present invention:

- the alphabet A is the set of the 20 amino acids;

- the set of $S_1,..., S_k$ input chains is formed by a plurality of protein sequences obtained from patients infected with a disease which vaccine is to be designed; and,
- the T set of target chains is formed by a set of epitopes.

Each feasible solution of the problem, that is to say, each λ-superchain obtained represents a possible vaccine, where λ indicates a lower limit of the number of different epitopes the vaccine should cover in each patient. Then, for a given λ, the optimal v solution of the problem represents the shortest vaccine, although in practice, the solutions of length closest to the shortest one are also of interest.

But the value between the optimal v and λ solution has to be compensated. On the one hand, the highest λ value corresponds to a better vaccine, since it covers a higher number of epitopes in each patient. On the other hand, the vaccine may only be effective if it is not too big (a too big vaccine may elicit an autoimmune response).

For this reason, in designing a vaccine, the problem of the shortest λ-superchain is solved several times, for different λ values, thus obtaining in each case the chain of the shortest possible length obtainable by the optimization method used. From among all the solutions obtained (optimal or approximate), as a general rule, those which allow for a better coverage of the epitope are preferred (typically, this corresponds to the highest values of λ). If λ is not high enough, then there is a good probability for other patients (not tested) to have a good percentage of the epitopes covered by the same vaccine. At the same time, of course, other biological considerations when determining the viability of a vaccine candidate represented by a λ-superchain, such as preserving the folding in the resulting pseudoproteins, should be taken on account.

The λ-superchain problem is a computationally difficult problem.

For this reason, in order to solve it, two different combinatorial optimization approaches have been used: solving by integer programming, which provides an optimal solution, and solving by a hill climbing algorithm, which provides a suboptimal solution.

In a real application example, vaccines for different λ values starting from a set of 123 sequences of patients infected with the H5N1 flu virus were obtained by means of the computational design method of the invention.

This set of sequences was chosen because Gilles and Ross (cf. Ref1) managed to find an effective vaccine protecting mice and ferrets against H5N1 by means of its computationally optimized broadly reactive antigen (COBRA), which opened new perspectives in the study of this type of synthetic vaccines. In designing their vaccine, they started with 129 input sequences from humans infected with clade 2 virus.

The reason why in this example not all the 129 sequences have been used is because although not all of them have the same length (in fact, it would be highly unusual if all of them had the same length), six of the sequences had significantly shorter lengths than the other sequences, and were not included in the calculations in order to work with high values of the λ parameter.

In the following Table 1 the identification number, ID, in the GenBank database of the 123 sequences used corresponding to haemagglutinin (HA) of the infected patients is included.

**Table 1**

| | | | | | | |
|---|---|---|---|---|---|---|
| EU146737 | EU146688 | CY014203 | CY014457 | EU015407 | FJ492886 | FJ492881 |
| EU146753 | EU146713 | CY014205 | CY014518 | EU015408 | EF200512 | HM114537 |
| EU146745 | EU146697 | CY014206 | CY014510 | EU015409 | EF200513 | FJ492880 |
| EU146793 | EU146705 | CY014207 | CY014481 | EU015410 | DQ464377 | |
| EU146755 | EU146729 | CY014209 | CY014489 | EU015411 | EU095023 | |
| EU146785 | EF541394 | CY014210 | CY014497 | EU015412 | EU095024 | |
| CY014272 | EU146777 | CY014211 | CY014177 | EU015413 | EU146867 | |
| CY014280 | EU146801 | CY014212 | CY014529 | EU015414 | EU146868 | |
| CY014288 | EU146809 | CY014213 | CY014543 | EU015416 | DQ371928 | |
| CY014296 | EU146817 | CY014311 | CY017662 | CY062439 | DQ371929 | |
| CY014303 | EU146825 | CY014368 | CY017670 | DQ435202 | EF624256 | |
| CY014477 | EU146632 | CY014376 | CY017678 | EU146870 | DQ835313 | |
| CY014433 | CY014197 | CY014384 | CY017688 | EU146876 | FJ492882 | |
| CY014465 | CY014160 | CY014393 | CY017638 | EU146877 | FJ492884 | |

(continued)

| EU146648 | CY014198 | CY014401 | CY017646 | EU146869 | DQ371930 | |
| EU146640 | CY014199 | CY014409 | CY017654 | EU146878 | EU263981 | |
| EU146656 | CY014200 | CY014417 | EU015403 | EF619982 | FJ492879 | |
| EU146664 | CY014201 | CY014425 | EU015404 | EF619989 | FJ492885 | |
| EU146672 | CY014204 | CY014441 | EU015405 | EF619990 | AB462295 | |
| EU146681 | CY014202 | CY014449 | EU015406 | EF619998 | EF137706 | |

Haemagglutinin has been selected in this example since Gilles and Ross demonstrated the effectiveness of a vaccine against clade 2 of H5N1 (avian flu) based on the epitopes of said protein.

One or several proteins may be used for each possible disease, although obviously that protein for which the vaccine is most effective is the one ultimately selected. In general, proteins with wide well conserved zones are used in each case, that is to say, with the lowest mutation indexes possible.

A hill climbing algorithm is used for this set of 123 sequences with 10000 iterations for $\lambda$ parameter values taken in steps of 10 from $\lambda$=10 to $\lambda$=500, where the alphabet A is the set of the 20 amino acids, and taking T=$A^{10}$ as the set of target epitopes. All the chains having a length between eight and fifteen are taken within the T set of target epitopes.

This approach has been chosen because, although the approach by integer programming provides optimal results, if said T set of epitopes formed by all the chains of a determined length is used, the computational cost is too high.

However, there exists the possibility of limiting the T set of epitopes by other biological criteria, such as the one already mentioned of preserving the folding in the obtained pseudoproteins, or selecting them from suitable epitope databases (such as, for example, "immune epitope database and analysis resource").

In Table 2 the lengths of the $\lambda$-superchains obtained in this case and their coverage (obtained according to equation [1]), which is the value in brackets in the table, are displayed.

**Table 2**

| | | |
|---|---|---|
| $\lambda$=10 : 19 (0.018) | $\lambda$=210 : 263 (0.417) | $\lambda$=410 : 729 (0.835) |
| $\lambda$=20 : 29 (0.036) | $\lambda$=220 : 273 (0.434) | $\lambda$=420 : 773 (0.837) |
| $\lambda$=30 : 39 (0.054) | $\lambda$=230 : 298 (0.453) | $\lambda$=430 : 790 (0.854) |
| $\lambda$=40 : 49 (0.073) | $\lambda$=240 : 319 (0.493) | $\lambda$=440 : 820 (0.872) |
| $\lambda$=50 : 64 (0.099) | $\lambda$=250 : 337 (0.514) | $\lambda$=450 : 861 (0.884) |
| $\lambda$=60 : 79 (0.125) | $\lambda$=260 : 356 (0.534) | $\lambda$=460 : 914 (0.906) |
| $\lambda$=70 : 89 (0.143) | $\lambda$=270 : 370 (0.558) | $\lambda$=470 : 973 (0.929) |
| $\lambda$=80 : 99 (0.161) | $\lambda$=280 : 390 (0.585) | $\lambda$=480 : 1031 (0.937) |
| $\lambda$=90 : 109 (0.179) | $\lambda$=290 : 406 (0.609) | $\lambda$=490 : 1095 (0.953) |
| $\lambda$=100: 119 (0.198) | $\lambda$=300 : 422 (0.62) | $\lambda$=500 : 1153 (0.958) |
| $\lambda$=110 : 129 (0.215) | $\lambda$=310 : 436 (0.64) | |
| $\lambda$=120: 139 (0.234) | $\lambda$=320 : 461 (0.679) | |
| $\lambda$=130 : 156 (0.252) | $\lambda$=330 : 483 (0.698) | |
| $\lambda$=140 : 166 (0.27) | $\lambda$=340 : 504 (0.717) | |
| $\lambda$=150 : 176 (0.288) | $\lambda$=350 : 523 (0.71) | |
| $\lambda$=160 : 190 (0.311) | $\lambda$=360 : 545 (0.735) | |
| $\lambda$=170 : 210 (0.338) | $\lambda$=370 : 574 (0.774) | |
| $\lambda$=180 : 226 (0.353) | $\lambda$=380 : 600 (0.785) | |
| $\lambda$= 190 : 242 (0.381) | $\lambda$=390 : 650 (0.807) | |

(continued)

| λ=200 : 252 (0.399) | λ=400 : 691 (0.826) | |
|---|---|---|

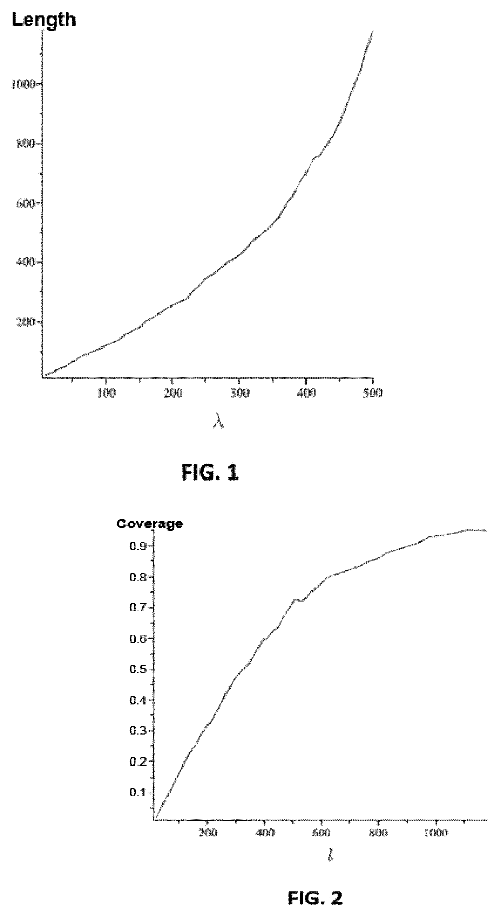

In Figures 1 and 2, the lengths and coverage obtained, respectively, have been plotted. As may be appreciated, the yield of the λ-superchain is higher for smaller λ values, in the sense that for relatively small λ values -about 360- the length of the λ-superchain (of the immunogen candidate for the vaccine) is also relatively small and is kept below the average length of haemagglutinin, which is 559.9 for the set of 123 sequences, and at the same time, the coverage is high, above 73 % of epitopes. As λ increases, the yield of the λ-superchain is not that good, because although it increases coverage (which is desirable), it also increases length, which may be troublesome since it could elicit an autoimmune response. In any case, even for a λ value of 490, the length is less than twice the average length of the protein and coverage is 95 %.

It is also possible to use genetic algorithms for obtaining a short length λ-superchain, such as the genetic algorithm described below, valid for high λ values. The fitness function used to assess the suitability of a vaccine candidate v is the number f(v) defined as the maximum value of λ for which each amino acid chain between $S_1,...,S_k$ contains at least λ epitopes of v, that is to say, f(v) is the maximum λ value for which the candidate v is already a λ-superchain (the maximum value is taken because it is of interest that λ is large with a small length of vaccine so that the vaccine covers many epitopes in each chain).

The genetic algorithm comprises:

- Prefixing a n number of epitopes in the vaccine, a s size of the initial population of vaccine candidates, a m number of evolution steps, a pr_cross crossing probability and a pr_mut mutation probability.
- Generating an initial population of s vaccine candidates wherein each member is obtained randomly concatenating an adequate number of chains selected among $S_1,...,S_k$ until obtaining s epitopes.
- Selecting s vaccine candidates from the population based on their suitability and applying crossover and mutation operations with respective pr_cross and pr_mut probabilities, and replacing the population with the new obtained candidates.
- Repeating the above process m times, and finally take the w candidate with better suitability obtained in the m evolution steps.
- Obtaining a common superchain having a next to the shortest length of the epitopes forming part of w, that is to say, a chain that accepts as superchains the epitopes found in the four previous steps and with length next to the shortest possible, by a greedy algorithm.

Example 1

In a first specific example of the procedure of the invention, a vaccine candidate against H5N1 virus was obtained using the genetic algorithm previously described for λ=335, and with the set of chains indicated in Table 1 taking haemagglutinin as protein. In this example 1 the T set of epitopes used as starting point is formed by all the sub-chains of length 10 of $S_1,...,S_k$. The amino acid sequence of the vaccine candidate obtained is as follows (provided as SEQ ID NO: 1):

FFWTILKPNDAINFESNGNFIAPEYAYKIVKKGDSAITIKRSYNNTNQEDLLVLW GIHHPNDAAEQTRLYQNPTTYISFFRNVVWLIKKEYGNCNTKCQTPTYDYPQY SEEARLKREEISGVKLESIGTYQILSIYSTVASSLALAIMLCYPGNFNDYEELKHL

LSRINHFEKIQIIPKSSWSDHEASSGVSSACPYLLAIVSLVKSDQICIGTSTLNQR
LVPKIATRSKVNGQSGRLVLATGLRNSPAGLSLWMCSNGSLQCRIGAINSSMP
FHNIHPLTIGECPKYVKSNHANNSTEQVDTIMEKNVTVTHAQDILEKTHNGKLC
DLDGVKPLILRDCSVAGWLLGNPMCDEFINVPEWSYIVEKANPANRKKRGLFG
AIAGFIEGGWQGMVDGWYGYHHSNEQGSGYAADKESTQKAIDGVTNKVNSII
DKMNTQFEAVGREFNNLERRIENLNKKMEDGFLDVWTYNAELLVLMENERTL
DFHDSNVKNLYDKVRLQLRDNAKELGNGCFEFYHKCDNECMES

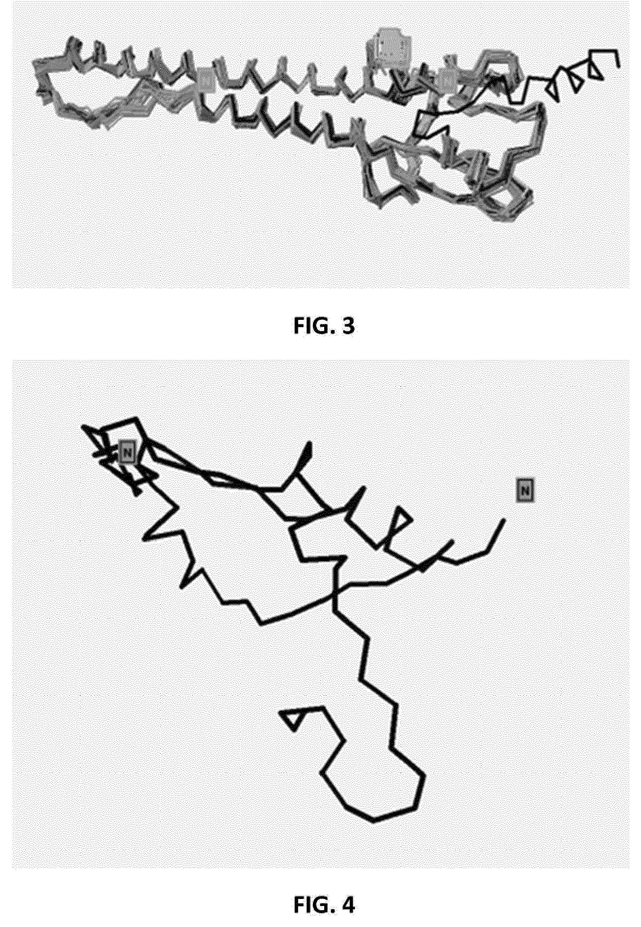

The structure of this vaccine candidate amino acid chain has been analyzed at the webpage http://www.proteinmodelportal.org/, having found the usual three-dimensional structures - alpha helices, beta sheets, etc. corresponding to haemagglutinin-. In Figure 3 an image of the vaccine candidate with a three dimensional model obtained in said webpage is shown; as can be seen, this structure corresponds to a vaccine one, and is not obtainable by any amino acid sequence.

Example 2

In a second specific example of the procedure of the invention, a vaccine candidate against HIV was obtained using the genetic algorithm previously described for λ=51, and taking NEF as protein. In this example 2 the T set of epitopes used as starting point is formed by all the sub-chains of length 9 of $S_1,...,S_k$.

In the following Table 3 the identification number, ID, in the GenBank database of the 169 sequences used corresponding to the protein NEF of the infected patients is included.

**Table 3**

| | | | | | | | | |
|---|---|---|---|---|---|---|---|---|
| AB012824 | AF120887 | AF129375 | AF203172 | AF238268 | AY121441 | AY835772 | L15515 | U34603 |
| AB034257 | AF120898 | AF129376 | AF203180 | AF252897 | AY173951 | AY835776 | L15518 | U43106 |
| AB034272 | AF120909 | AF129377 | AF203188 | AF252910 | AY308762 | AY835779 | M17451 | U44444 |
| AB078005 | AF129334 | AF129378 | AF203192 | AF462708 | AY314063 | AY835780 | M21098 | U44450 |
| AB221005 | AF129335 | AF129379 | AF203194 | AF462753 | AY331285 | AY857022 | M26727 | U44462 |
| AF004394 | AF129342 | AF129382 | AF203198 | AF538302 | AY331290 | AY857144 | M58173 | U44468 |
| AF011471 | AF129343 | AF129388 | AF219672 | AF538304 | AY331293 | AY899356 | M93259 | U66543 |
| AF011474 | AF129346 | AF129389 | AF219685 | AF538305 | AY352275 | AY899382 | U03295 | U69584 |
| AF011481 | AF129347 | AF129390 | AF219691 | AF538306 | AY444311 | DQ007902 | U03338 | U71182 |
| AF011487 | AF129350 | AF129392 | AF219729 | AJ271445 | AY713408 | DQ085869 | U03343 | |
| AF011493 | AF129351 | AF129394 | AF219755 | AJ430664 | AY739040 | DQ121815 | U12055 | |
| AF042101 | AF129352 | AF203108 | AF219760 | AY037269 | AY779550 | DQ121883 | U16863 | |
| AF047082 | AF129354 | AF203111 | AF219765 | AY037282 | AY786630 | DQ127537 | U16875 | |
| AF063926 | AF129355 | AF203116 | AF219771 | AY116676 | AY786750 | DQ127548 | U16934 | |
| AF069139 | AF129362 | AF203126 | AF219782 | AY116713 | AY835748 | DQ487191 | U23487 | |
| AF120745 | AF129364 | AF203137 | AF219792 | AY116714 | AY835751 | DQ659737 | U24455 | |
| AF120772 | AF129369 | AF203141 | AF219800 | AY116727 | AY835753 | L07422 | U26087 | |
| AF120840 | AF129370 | AF203153 | AF219812 | AY116781 | AY835762 | L15482 | U26110 | |
| AF120851 | AF129372 | AF203161 | AF219819 | AY116805 | AY835765 | L15489 | U26119 | |
| AF120867 | AF129373 | AF203165 | AF219845 | AY116830 | AY835770 | L15500 | U26138 | |

The amino acid sequence of the vaccine candidate obtained is as follows (provided as SEQ ID NO: 2):

LGWCFKLVPVEPDKVEEANKGENNCLLHPMSQHGMAVRERMRRAEPAADG
VGAVSRDLEKHGAITSSLAFHHVARELHPEVLMWKFDSRLAFHHMARELHPE
YYCAWLEAQEEEVGFPVKPKGALDLSHFLKEKGGLEGLIYSQKRQDILDLWV
YHTQGYFPDWQNYTPGPGIRYPLTFGWCFKLVPVEPEKIYHTQGYFPAWLEA
QEDEEVGFPNNSLLHPMSLHGEVGFPVKPQVPLRPMEEEVGFPVRPQVPLR
PMTYKAAVDCWKLVPVEPDGVGAASRDLEKHGWSTVRERMRRAREGENNS
LLHPI

In Figure 4 an image of the three dimensional structure of this vaccine candidate is displayed.

Example 3

In a third specific example of the procedure of the invention, a vaccine candidate against HIV was obtained using the genetic algorithm previously described for λ=140, and taking GAG as protein. As in example 2, in this example 3 the T set of epitopes used as starting point is formed by all the sub-chains of length 9 of the $S_1,...,S_k$.

In the following Table 4 the identification number, ID, in the GenBank database of the 166 sequences used corresponding to the protein (GAG) of the infected patients is included.

**Table 4**

| AB078005 | AF146728 | AY173951 | AY206663 | AY560108 | AY786962 | AY835776 | DQ295192 | U34604 |
|---|---|---|---|---|---|---|---|---|
| AB078703 | AF224507 | AY173952 | AY206664 | AY560110 | AY818644 | AY835777 | DQ295193 | U39362 |
| AB078704 | AF256204 | AY173954 | AY247251 | AY679786 | AY819715 | AY835778 | DQ295195 | U43096 |
| AB078709 | AF286365 | AY173955 | AY275555 | AY682547 | AY835748 | AY835779 | DQ487188 | U43141 |
| AB078711 | AF538302 | AY173956 | AY275556 | AY751406 | AY835751 | AY835780 | DQ487189 | U69584 |
| AB097870 | AF538303 | AY180905 | AY275557 | AY751407 | AY835753 | AY839827 | DQ487190 | U71182 |
| AB221005 | AF538304 | AY206647 | AY308760 | A779550 | AY835754 | AY857022 | DQ487191 | |
| AF003887 | AF538305 | AY206648 | AY308762 | AY779553 | AY835755 | AY857144 | K02007 | |
| AF004394 | AF538306 | AY206649 | AY314044 | AY779556 | AY835757 | AY970946 | L02317 | |
| AF042100 | AF538307 | AY206651 | AY314063 | AY779557 | AY835758 | AY970950 | M13136 | |
| AF042101 | AJ271445 | AY206652 | AY331283 | AY779563 | AY835759 | CQ958304 | M17451 | |
| AF042102 | AJ437030 | AY206653 | AY331285 | AY779564 | AY835761 | D10112 | M19921 | |
| AF042103 | AJ437033 | AY206654 | AY331287 | AY786790 | AY835762 | DQ085869 | M26727 | |
| AF042104 | AJ437038 | AY206656 | AY331290 | AY786830 | AY835764 | DQ097739 | M38429 | |
| AF042105 | AJ437039 | AY206657 | AY331292 | AY786870 | AY835765 | DQ097744 | M38431 | |
| AF049494 | AJ437044 | AY206658 | AY331297 | AY786910 | AY835766 | DQ097747 | M93258 | |
| AF049495 | AJ437047 | AY206659 | AY332236 | AY786919 | AY835769 | DQ127536 | U21135 | |
| AF069140 | AJ437050 | AY206660 | AY352275 | AY786920 | AY835770 | DQ127539 | U23487 | |
| AF075719 | AJ437051 | AY206661 | AY423387 | AY786949 | AY835772 | DQ127542 | U26546 | |
| AF086817 | AJ437058 | AY206662 | AY560107 | AY786952 | AY835774 | DQ127548 | U34603 | |

The amino acid sequence of the vaccine candidate obtained in this case is as follows (provided as SEQ ID NO: 3):

YKRWIILGLNKIVRMYSPMGARASVLSGGELDQKQEPIDKEQAAMQMLKETIN
DQMLKETINEEAAEWDRLHPVHAGPIAPGQMREPRGSDIAGTTSTLQEQIGW
MTATLEEMMTACQGVGGPGHKARVLAEAMSQVTNSARNCRAPRKKGCWKC
GKEGHQMKDCTERQANFLGKIWPSHKGRPGNFLQSRPEPTAPPEESILDIRQ
GPKEPFRDYVDRFYKTLRAEQASQEVKNWMTETLLVQNANPDCKTILKALGP
RLRPGGKKKYKLKHIVWASRELERFAVNPGLLETSWKCGQEGHQLQPSLQT
GSEELKSLYYTVATLYCVHQRIEVEDTKEALDKIEEEQNKSNKKAHQAEAAAD

TGHNKQVSQNYPIVQNLQGQMVHQPISPRTLNAWVKVVEEKAFSPEVIPMFS
ALAEGATPQDLNTMLNTVGDIKQPCRCVKCFNCGKEGH

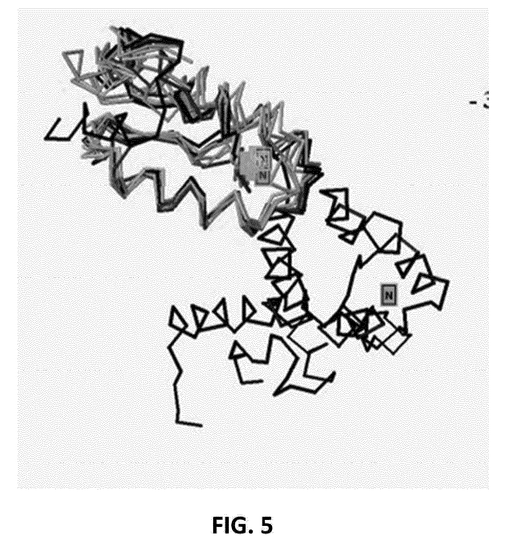

In Figure 5 an image of the three dimensional structure of this vaccine candidate is displayed.

It is important to take into account that non-optimal solutions also have relatively short lengths and good coverage levels, and at the same time, given the combinatorial properties taken into account during its production, they pose an adequate balance of epitopes in the set of sequences chosen from infected patients.

In fact, a key indicator of the antigenic potential of a vaccine is determined by the percentage of covered epitopes. The antigenic effectiveness of a vaccine depends, at the same time, that there is no, or in any case there is the minimum number of patient with epitopes not recognizable by the vaccine. The procedure of the invention by obtaining the $\lambda$-superchain guarantees accomplishing this second objective and also, indirectly and as a consequence derived from its properties, accomplishing the first high percentage of covered epitopes objective.

In this text, the word "comprises" and its variants (such as "comprising", etc.) should not be construed in an exclusive manner, that is to say, they do not exclude the possibility that what is described includes other elements, steps, etc.

On the other hand, the invention is not limited to the specific embodiments described but it also encompasses, for example, the variants which may be performed by one of ordinary skill in the art (for example, with regard to the choice of materials, dimensions, components, configuration, etc.), within what is deduced from the claims.

**Claims**

1.    Computational procedure for designing a vaccine for a disease that starting:

    - from a plurality of amino acid $S_1$,...,$S_k$ chains obtained from patients infected with such disease; and,
    - from a T set of target epitopes, each epitope being an aminoacid chain; comprises the steps of:

        i) obtaining by means of a combinatory optimization method a plurality of amino acid chains as short as possible with respect to the combinatory optimization method used having as sub-chains at least a $\lambda$ integer of epitopes of the T set in each one of the $S_i$ chains, $\lambda$ having a value such that the length of the resulting chain is long enough for eliciting an immune response, but not too long so as to elicit an autoimmune response;
        ii) choosing from the plurality of chains obtained in the previous step at least one final immunogen candidate by at least one standard experimental method;
        iii) developing the vaccine from said immunogen.

2.    Procedure according to claim 1, wherein the combinatorial optimization method is integer programming.

3.    Procedure according to claim 1, wherein the combinatorial optimization method is a hill climbing algorithm.

4.    Procedure according to claim 1, wherein the combinatorial optimization method is a genetic algorithm.

5.    Procedure according to claim 4, wherein the genetic algorithm comprises:

    - prefixing a n number of epitopes in the vaccine, a s size of the initial population of vaccine candidates, a m number of evolution steps, a pr_cross crossing probability and a pr_mut mutation probability;

- generating an initial population of s vaccine candidates wherein each member is obtained randomly concatenating an adequate number of chains selected among $S_1,...,S_k$ until obtaining s epitopes;

- selecting s vaccine candidates from the population based on their suitability and applying crossover and mutation operations, and replacing the population with the new candidates obtained;

- repeating the above process m times, and finally take the w candidate with better suitability obtained in the m evolution steps;

- obtaining a common superchain, with a next to the shortest length, of the epitopes belonging to w by a greedy algorithm.

6. Procedure according to claim 1, wherein the combinatorial optimization method is a simulated annealing algorithm.

7. Procedure according to claim 1-6, wherein for each vaccine to be designed, such $S_1,...,S_k$ amino acid chains correspond to the same protein for all infected patients.

8. Procedure according to claim 1-6, wherein for each vaccine to be designed, as $S_1,...,S_k$ amino acid chains two or more proteins from infected patients are used.

9. Procedure according to any of claims 1-8, wherein said T set of epitopes is formed by all the chains of a determined length.

10. Procedure according to any of claims 1-8, wherein said T set of epitopes is empirically obtained.

11. Procedure according to any of claims 1-8, wherein said T set of epitopes is obtained from a database of epitopes.

12. Procedure according to any of claims 1-11, wherein said standard experimental method includes applying immunogenicity tests.

13. Procedure according to any of claims 1-12, wherein said plurality of $S_1,...,S_k$ amino acid chains is obtained from a database of biological sequences.

14. Procedure according to any of claims 1-13, wherein the steps i)-iii) are repeated for different $\lambda$ values and the one providing the highest epitope coverage is chosen from the solutions obtained.

**Length**

**FIG. 1**

**Coverage**

**FIG. 2**

FIG. 3

FIG. 4

FIG. 5

## INTERNATIONAL SEARCH REPORT

| International application No |
|---|
| PCT/ES2014/070888 |

**A. CLASSIFICATION OF SUBJECT MATTER**

INV. A61K39/00    G06F19/00
ADD.

According to International Patent Classification (IPC) or to both national classification and IPC

**B. FIELDS SEARCHED**

Minimum documentation searched (classification system followed by classification symbols)

A61K  G06F

Documentation searched other than minimum documentation to the extent that such documents are included in the fields searched

Electronic data base consulted during the international search (name of data base and, where practicable, search terms used)

EPO-Internal, BIOSIS, EMBASE, WPI Data

**C. DOCUMENTS CONSIDERED TO BE RELEVANT**

| Category* | Citation of document, with indication, where appropriate, of the relevant passages | Relevant to claim No. |
|---|---|---|
| X | NORA C. TOUSSAINT ET AL: "A Mathematical Framework for the Selection of an Optimal Set of Peptides for Epitope-Based Vaccines", PLOS COMPUTATIONAL BIOLOGY, vol. 4, no. 12, 26 December 2008 (2008-12-26), page e1000246, XP055181707, DOI: 10.1371/journal.pcbi.1000246 page 1, right-hand column, paragraph 2 - page 2, right-hand column, paragraph 2; tables 1-3 ----- -/-- | 1-14 |

[X] Further documents are listed in the continuation of Box C.    [X] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 16 April 2015 | 22/04/2015 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Cilensek, Zoran |
|---|---|

Form PCT/ISA/210 (second sheet) (April 2005)

## INTERNATIONAL SEARCH REPORT

International application No.

PCT/ES2014/070888

| Box No. I | Nucleotide and/or amino acid sequence(s) (Continuation of item 1.c of the first sheet) |

1.     With regard to any nucleotide and/or amino acid sequence disclosed in the international application, the international search was carried out on the basis of a sequence listing:

    a.  ☐  forming part of the international application as filed:

        ☐  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☒  furnished subsequent to the international filing date for the purposes of international search only:

        ☒  in the form of an Annex C/ST.25 text file (Rule 13*ter*.1(a)).

        ☒  on paper or in the form of an image file (Rule 13*ter*.1(b) and Administrative Instructions, Section 713).

2.  ☒  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet (1)) (January 2015)

## INTERNATIONAL SEARCH REPORT

International application No
PCT/ES2014/070888

| C(Continuation). DOCUMENTS CONSIDERED TO BE RELEVANT | | |
|---|---|---|
| Category* | Citation of document, with indication, where appropriate, of the relevant passages | Relevant to claim No. |
| X | WILL FISCHER ET AL: "Polyvalent vaccines for optimal coverage of potential T-cell epitopes in global HIV-1 variants", NATURE MEDICINE, NATURE PUBLISHING GROUP, NEW YORK, NY, US, vol. 13, no. 1, 1 January 2007 (2007-01-01), page 100, XP007911386, ISSN: 1078-8956, DOI: 10.1038/NM1461 page 101, right-hand column, paragraph 3 page 102, right-hand column, paragraph 3 - page 104, right-hand column, paragraph 1 ----- | 1-14 |
| X,P | LUIS MARTÍNEZ ET AL: "A combinatorial approach to the design of vaccines", JOURNAL OF MATHEMATICAL BIOLOGY, vol. 70, no. 6, 25 May 2014 (2014-05-25), pages 1327-1358, XP055181425, ISSN: 0303-6812, DOI: 10.1007/s00285-014-0797-4 the whole document ----- | 1-14 |
| A | BRENDAN M GILES ET AL: "A computationally optimized broadly reactive antigen (COBRA) based H5N1 VLP vaccine elicits broadly reactive antibodies in mice and ferrets", VACCINE, ELSEVIER LTD, GB, vol. 29, no. 16, 29 January 2011 (2011-01-29), pages 3043-3054, XP028160611, ISSN: 0264-410X, DOI: 10.1016/J.VACCINE.2011.01.100 [retrieved on 2011-02-04] the whole document ----- | 1-14 |
| A | WO 2008/157530 A2 (MICROSOFT CORP [US]) 24 December 2008 (2008-12-24) the whole document ----- | 1-14 |

Form PCT/ISA/210 (continuation of second sheet) (April 2005)

## INTERNATIONAL SEARCH REPORT
Information on patent family members

International application No

PCT/ES2014/070888

| Patent document cited in search report | Publication date | Patent family member(s) | Publication date |
|---|---|---|---|
| WO 2008157530 A2 | 24-12-2008 | US 2008312095 A1<br>WO 2008157530 A2 | 18-12-2008<br>24-12-2008 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **D.C. NICKLE et al.** Coping with viral diversity in HIV vaccine design. *PLoS Comput. Biol.,* 2007, vol. 3 (e75), 0754-0762
- **FISHER W. et al.** Polyvalent vaccines for optimal coverage of potential T-cell epitopes in global HIV-1 variants. *Nature Medicine,* 2007, vol. 13 (1), 100106
- **TOUSSAINT N.C. et al.** A mathematical framework for the selection of an optimal set of peptides for epitope-based vaccines. *PLoS Comput. Biol.,* 2008, vol. 4, 1-10
- **GILLES B.M. et al.** A computationally optimized broadly reactive antigen (COBRA) based H5N1 VLP vaccine elicits broadly reactive antibodies in mice and ferrets. *Vaccine,* 2011, vol. 29, 3043-3054
- **KULKARNI et al.** HIV-1 p24gag derived conserved element DNA vaccine increases the breadth of immune response in mice. *PLoS one,* 2013, vol. 8 (3), e60245